# FASCICULE DE BREVET EUROPEEN

(11) **EP 0 678 023 B1**
(45) Date de publication et mention de la délivrance du brevet: **23.10.1996**
(21) Numéro de dépôt: 94903935.8
(22) Date de dépôt: 03.01.1994
(51) Int. Cl.: A61K 31/425

(54) **APPLICATION DU RILUZOLE DANS LE TRAITEMENT DE LA MALADIE DE PARKINSON ET DES SYNDROMES PARKINSONIENS**
VERWENDUNG VON RILUZOL ZUR BEHANDLUNG VON PARKINSON UND DEN PARKINSON-SYNDROMEN
APPLICATION OF RILUZOLE IN THE TREATMENT OF PARKINSON'S DISEASE AND PARKINSONIAN SYNDROMES

(30) Priorité: 07.01.1993 FR 9300074
(43) Date de publication de la demande: 25.10.1995
(73) Titulaire: RHONE-POULENC RORER S.A., 92160 Antony (FR)
(72) Inventeur: BOIREAU, Alain, F-94370 Sucy-en-Brie (FR); DOBLE, Adam, F-75005 Paris (FR); DUBEDAT, Pierre, F-94130 Nogent-sur-Marne (FR); LOUVEL, Erik, F-75010 Paris (FR); MEUNIER, Mireille, F-91410 Dourdan (FR); MIQUET, Jean-Marie, F-91400 Orsay (FR); STUTZMANN, Jean-Marie, F-94440 Villecresnes (FR)
(74) Mandataire: Savina, Jacques
(86) Numéro de dépôt international: FR9400003
(87) Numéro de publication internationale: WO9415601

(56) Documents cités:
- EP-A- 0 050 551
- EP-A- 0 517 347
- THE JOURNAL OF NEUROSCIENCE vol. 9, no. 11 , 1989 pages 3720 - 3727 C. MALGOURIS ET AL. 'RILUZOLE, A NOVEL ANTIGLUTAMATE, PREVENTS MEMORY LOSS AND HIPPOCAMPAL NEURONAL DAMAGE IN ISCHAEMIC GERBILS'
- THE JAPANESE JOURNAL OF PHARMACOLOGY vol. 49, no. SUPL , 1989 page 8P J.C. BLANCHARD ET AL. 'NEUROPROTECTION AGAINST ISCHAEMIA AND IMPROVEMENT OF SLEEP QUALITY WITH RILUZOLE A NOVEL ANTIGLUTAMATE'
- NEUROSCIENCE LETTERS vol. 140, no. 2 , 22 Juin 1992 pages 225 - 230 J. PRATT ET AL. 'NEUROPROTECTIVE ACTIONS OF RILUZOLE IN RODENT MODELS OF GLOBAL AND FOCAL CEREBRAL ISCHAEMIA'
- NEUROPHARMACOLOGY vol. 24, no. 11 , 1985 pages 1085 - 1092 J. BENAVIDES ET AL. '2-AMINO-6-TRIFLUOROMETHOXY BENZOTHIAZOLE, A POSSIBLE ANTAGONIST OF EXCITATORY AMINO ACID NEUROTRANSMISSION-II'
- NEUROSCIENCE LETTERS vol. 147, no. 2 , 7 Décembre 1992 pages 209 - 212 A. CHERAMY ET AL. 'RILUZOLE INHIBITS THE RELEASE OF GLUTAMATE IN THE CAUDATE NUCLEUS OF THE CAT IN VIVO'
- TRENDS IN NEUROSCIENCE vol. 12, no. 8 , 1989 pages 285 - 286 T. KLOCKGETHER 'EXCITATORY AMINO ACIDS AND THE BASAL GANGLIA: IMPLICATIONS FOR THE THERAPY OF PARKINSON'S DISEASE'
- The Journal of Neuroscience, vol 7 (10), 1987, pages 3343-3349
- Journal of Neurochemistry, vol. 58 (5), 1992, pages 1979-1982
- Brain Research, vol. 592, 1992, pages 74-83
- European Journal of Pharmacology, vol. 235, 1993, pages 297-300
- J. Neural Transm., vol. 3, 1991, pages 203-213
- Journal of Neurochemistry, vol. 65 (2), 1995, pages 652-659
- J. Neural Transm. (Suppl.), vol. 43, 1994, pages 81-89

## Description

La présente invention concerne une nouvelle application thérapeutique du riluzole (trifluorométhoxy-6 amino-2 benzothiazole) ou les sels pharmaceutiquement acceptables de ce composé.

Le riluzole est utile comme médicament anticonvulsivant, anxiolytique et hypnotique (brevet EP 50551), dans le traitement de la schizophrénie (EP 305276), dans le traitement des troubles du sommeil et de la dépression (EP 305277), dans le traitement des désordres cérébrovasculaires et comme anesthésique (EP 282971).

Il est aussi connu que le riluzole s'oppose à la dégénérescence des cellules pyramidales de l'hippocampe lors d'une ischémie cérébrale (The Journal of Neuroscience 9(11), pages 3720 - 3727, 1989).

Il a maintenant été trouvé de manière surprenante que ce composé peut aussi être utilisé dans le traitement de la maladie de Parkinson et des syndromes parkinsoniens.

Il est connu que la neurotoxine MPTP (1-méthyl-4-phényl-1,2,3,6-tétrahydropyridine) induit un syndrome similaire à la maladie de Parkinson. Ce syndrome résulte d'une dégénération des neurones nigrostriataux dopaminergiques chez les primates (R. S. BURNS et coll., Proc. Natl. Acad. Sci., 80, 4546-4550 (1983), chez l'homme (J. W. LANGSTON et coll., Science, 219, 979-980 (1983)) et chez la souris (R. E. HEIKKILA et coll., Science, 224, 1451-1453 (1984).

L'activité du riluzole a donc été mise en évidence chez la souris en mesurant les diminutions des taux de dopamine striatale et corticale induites par la MPTP comparées à celles des animaux témoins.

On injecte, 3 fois à 2 heures d'intervalle, 15 mg/kg de MPTP par voie intrapéritonéale à des souris (C57BL/6) pesant 20-25 g. Trente minutes avant la première injection de MPTP, puis 2 heures et 30 minutes, 5 heures et 30 minutes et 7 heures et 30 minutes après la première injection de MPTP on administre selon le produit de 1 à 40 mg/kg du produit à étudier. Durant les 3 jours suivants, on administre 2 fois par jour selon le produit de 1 à 40 mg/kg du produit à étudier. Les souris sont sacrifiées 8 jours après l'injection de MPTP. Le striatum et le cortex frontal sont disséqués et conservés à -70°C jusqu'au moment de leur analyse. Les taux de dopamine, sont mesurés par chromatographie liquide haute pression avec une détection électrochimique. Les analyses statistiques sont effectuées en utilisant ANOVA suivi par le test de SCHEFFE.

Les résultats obtenus sont mentionnés dans le tableau suivant :

| | taux de dopamine pmol/mg dans le striatum (% par rapport aux témoins) | taux de dopamine pmol/mg dans le cortex frontal (% par rapport aux témoins) |
|---|---|---|
| témoins | 876±64 | 2,486±0,290 |
| animaux ne recevant que de la MPTP | 219±18 (-75%) | 1,481±0,180 (-40%) |
| animaux traités avec le riluzole | 378±39 (-57%) | 2,359±0,185 (-5%) |

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale ou parentérale.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter. Les exemples suivants illustrent des médicaments selon l'invention :

### Exemple A

On prépare, selon la technique habituelle, des comprimés dosés à 50 mg de produit actif ayant la composition suivante :

| | |
|---|---|
| - Riluzole | 50 mg |
| - Mannitol | 64 mg |
| - Cellulose microcristalline | 50 mg |
| - Polyvidone excipient | 12 mg |
| - Carboxyméthylamidon sodique | 16 mg |
| - Talc | 4 mg |
| - Stéarate de magnésium | 2 mg |
| - Silice colloïdale anhydre | 2 mg |
| - Mélange de méthylhydroxypropylcellulose, polyéthylèneglycol 6000, dioxyde de titane (72-3,5-24,5) q.s.p. 1 comprimé pelliculé terminé à | 245 mg |

### Exemple B

On prépare, selon la technique habituelle, des gélules dosées à 50 mg de produit actif ayant la composition suivante :

| | |
|---|---|
| - Riluzole | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### Exemple C

On prépare une solution injectable contenant 10 mg de produit actif ayant la composition suivante :

| | |
|---|---|
| - Riluzole | 10 mg |
| - Acide benzoïque | 80 mg |
| - Alcool benzylique | 0,06 cm3 |
| - Benzoate de sodium | 80 mg |
| - Ethanol à 95 % | 0,4 cm3 |
| - Hydroxyde de sodium | 24 mg |
| - Propylène glycol | 1,6 cm3 |
| - Eau | q.s.p. 4 cm3 |

L'invention concerne également le procédé de préparation de médicaments utiles dans le traitement de la maladie de Parkinson et des syndromes parkinsoniens consistant à mélanger le riluzole ou les sels pharmaceutiquement acceptables de ce composé avec un ou plusieurs diluants et/ou adjuvants compatibles et pharmaceutiquement acceptables.

L'invention concerne également l'application d'une quantité efficace de riluzole ou des sels pharmaceutiquement acceptables de ce composé. à la péparation de médicaments destinés au traitement de la maladie Parkinson et des syndromes parkinsoniens chez un mannifère, notamment l'homme.

## Revendications

1. Application du riluzole ou les sels pharmaceutiquement acceptables de ce composé à la préparation de médicaments destinés au traitement de la maladie de Parkinson et des syndromes parkinsoniens.

2. Application selon la revendication 1 pour obtenir un médicament comprenant 25 à 200 mg de riluzole.

3. Procédé de préparation d'un médicament utile pour le traitement de la maladie de Parkinson et des syndromes parkinsoniens caractérisé en ce que l'on mélange le riluzole ou les sels pharmaceutiquement acceptables de ce composé avec un ou plusieurs diluants et/ou adjuvants compatibles et pharmaceutiquement acceptables.

## Claims

1. Application of riluzole or pharmaceutically acceptable salts of this compound to the preparation of medicinal products intended for the treatment of Parkinson's disease and parkinsonian syndromes.

2. Application according to claim 1, for obtaining a medicinal product comprising 25 to 200 mg of riluzole.

3. Process for preparing a medicinal product which can be used for the treatment of Parkinson's disease and parkinsonian syndromes, characterized in that riluzole or the pharmaceutically acceptable salts of this compound is/are mixed with one or more compatible and pharmaceutically acceptable diluents and/or adjuvants.

## Patentansprüche

1. Verwendung von Riluzol oder den pharmazeutisch annehmbaren Salzen dieser Verbindung zur Herstellung von Arzneimitteln für die Behandlung der Parkinson-Krankheit und von Parkinson-Syndromen,

2. Verwendung gemäß Anspruch 1, um ein Arzneimittel zu erhalten, das 25 bis 200 mg Riluzol enthält.

3. Verfahren zur Herstellung eines für die Behandlung der Parkinson-Krankheit und von Parkinson-Syndromen verwendbaren Arzneimittels, dadurch gekennzeichnet, daß man Riluzol oder die pharmazeutisch annehmbaren Salze dieser Verbindung mit einem oder mehreren verträglichen und pharmazeutisch annehmbaren Verdünnungsmitteln und/oder Adjuvantien mischt.
